# EUROPÄISCHE PATENTSCHRIFT

(11) **EP 1 689 415 B1**
(45) Veröffentlichungstag und Bekanntmachung des Hinweises auf die Patenterteilung: **29.12.2010**
(21) Anmeldenummer: 04803383.1
(22) Anmeldetag: 01.12.2004
(51) Int. Cl.: A61K 33/26, A61K 31/295, A61K 31/375, A23L 1/302, A23L 1/304, A61P 3/02

(54) **VERBESSERUNG DER EISENAUFNAHME**
IMPROVING IRON UPTAKE
AMELIORATION DE L'ABSORPTION DU FER

(30) Priorität: 05.12.2003 DE 10357277
(43) Veröffentlichungstag der Anmeldung: 16.08.2006
(73) Patentinhaber: Phyt-Immun GmbH, 66424 Homburg (DE)
(72) Erfinder: THEISS, Peter, 66424 Homburg (DE)
(74) Vertreter: Luderschmidt, Schüler & Partner
(86) Internationale Anmeldenummer: PCT/EP2004/013611
(87) Internationale Veröffentlichungsnummer: WO 2005/053715

(56) Entgegenhaltungen:
- WO-A-01/30352
- US-A- 5 160 728
- US-A1- 2002 197 330
- US-B1- 6 562 378

## Beschreibung

Die vorliegende Erfindung betrifft Präparate zur Verbesserung des menschlichen Wöhlbefindens, und insbesondere zur verbesserten Eisenversorgung beim Menschen. Es werden Verfahren zur Herstellung solcher Präparate offenbart. Insbesondere finden diese Präparate als ergänzende bilanzierte Diät Verwendung.

Eisen ist ein lebenswichtiges Spurenelement, das physiologisch als Zentralatom vor allem im Hämoglobinmolekül, in Myoglobin sowie in zahlreichen Enzymen vorkommt und eine Schlüsselrolle bei einigen Stoffwechselvorgängen, insbesondere bei der Sauerstoffversorgung der Organe, einnimmt.

Etwa 65 bis 85 % des gesamten Eisenbestandes sind im Hämoglobin der Erythrozyten und Erythrozytenvorstufen, weitere 10 bis 15 % des Eisenbestandes sind in einer Vielzahl eisenhaltiger Enzyme und in Form von Myoglobin im Muskelgewebe gebunden. Die restlichen durchschnittlich 0 bis 25 % des Eisenbestandes stellen funktionell inaktives Reserveeisen dar. Der Gesamteisenbestand beim Mann beträgt im Mittel 50 mg Eisen pro Kilogramm Körpergewicht, dies entspricht einer Gesamteisenmenge von 3500 mg bei einem 70 kg schweren Mann. Bei Frauen ist die Konzentration mit circa 38 mg Eisen pro kg Körpergewicht niedriger und entsprechend auch die Gesamtmenge von 2280 mg bei einer 60 kg schweren Frau (Baynes et al. 1994, Benn HP et al. 1993, Cook JD et al. 1991). Der Gesamteisenbestand kann durch Messung der. Serumferritinkonzentration abgeschätzt werden (1 µg/l Ferritin entspricht 8 mg Körpereisenmenge).

Eisenmangel ist in seinen verschiedenen Ausprägungsformen immer noch die häufigste oder am häufigsten übersehene oder fehlgedeutete Eisenstoffwechselstörung. Vermutlich ist er auch der häufigste ernährungsbedingte Mangelzustand überhaupt (M. Wick, 2000). Den Verlauf eines Eisenmangels kann man in 3 charakteristische Stadien unterteilen: prälatenter Eisenmangel = Speichereisenmangel mit erniedrigter Ferritinkonzentration im Serum sowie Eisenverarmung der Speicherorgane, latenter Eisenmangel mit Mangel an Speicher- und Transporteisen (niedriges Serumferritin und erniedrigte Transferrinsättigung) und manifester Eisenmangel = Eisenmangelanämie (mikrozytäre hypochrome Anämie). Anämiesymptome können sein: Schwäche, Müdigkeit, Leistungsknick, Kopfschmerzen, Schwindel, Belastungsdyspnoe, Tachykardie, Herzklopfen.

Es ist allgemein bekannt, dass Blutspender, Kinder und Jugendliche, menstruierende Frauen, Schwangere und Leistungssportler hinsichtlich der Entwicklung eines Eisenmangels besonders gefährdet sind.

Der Zusammenhang bei Dauerspendern wurde u.a. in einer Studie von Alvarez-Ossorio, L et al. (2000) bewiesen. Es wurde eine große Zahl von Probanden (632 Dauerspender und 171 Erstspender) untersucht. Die Gesamteisenspeichermenge war abhängig von der Spendenzahl pro Jahr. Der Ferritin-Spiegel sank bei Dauerspendern nach der 10. Spende signifikant und blieb bei den nächsten 20 oder mehr Spenden konstant. Bei 26 % der Dauerspender lag der Wert < 15 µg/l und bei 12 % kam es zu Entwicklung einer Anämie mit erniedrigten Hämoglobinwerten. Bei Ferritin < 15 µg/l wurde eine 20-tägige Eisensubstitution (100 mg/Tag) durchgeführt. Nach der Substitution kam es zu einer raschen Erholung der Hämoglobinwerte bei den Spendern mit anfänglich niedrigen Werten und eine anämiebedingte Zurückstellung von der Spende wurde nicht beobachtet. Die Dauerspender wurden in 5 Gruppen in Abhängigkeit von der jährlichen Spendenfrequenz unterteilt. Es zeigte sich auch ein signifikanter Unterschied zwischen dem Ferritin-Spiegel bei Erst- und Dauerspendern nach der 10. Spende. Diese Studie zeigt eine Notwendigkeit einer regelmäßigen Kontrolle des Eisenspeichers mittels Ferritinmessung bei Blutspendern und einer Eisensubstitution zur Prophylaxe eines chronischen Eisenmangels infolge regelmäßiger Blutspende.

Der Bedarf an Eisen richtet sich nach dem Verlust. Die täglichen Verluste über Gastrointestinaltrakt, Haut und Urin betragen circa 1 mg, bei menstruierenden Frauen kommen weiter 0,7 mg täglich hinzu. Bei Schwangerschaft wird 1 mg täglich zusätzlich benötigt (Linkesch et al. 1984, Siegenthaler, W et al. 1988).

Eisen ist ein Bestandteil der normalen Ernährung. Die resorbierbare Menge ist von den Ernährungsgewohnheiten abhängig. Am besten wird Eisen aus Fleischprodukten resorbiert. Mit 1000 Kcal einer mitteleuropäischen Mahlzeit werden ungefähr 6 mg absorbierbares Eisen zugeführt. Dies entspricht einer durchschnittlichen Zufuhr von 11 bis 17 mg Eisen pro Tag mit der Nahrung (Watson, WS et al 1986). Von dieser Menge können jedoch nur maximal 20 % resorbiert werden. Maximal können demnach Eisenmengen von 3,5 mg Eisen pro Tag bei normaler Ernährung resorbiert werden (Finch, CA et al. 1977). Um höhere Mengen resorbieren zu können, ist eine zusätzliche Eisensubstitution notwendig.

Möglicherweise begünstigen hohe Eisenspiegel die Entwicklung von atherosklerotischen Veränderungen der Gefäßwand (Sullivan, JL 1991). Eisensubstitutionen in Dosierungen über 100 mg/Tag sind daher normalerweise zu vermeiden.

Eisen wird im Duodenum und oberen Jejunum als Fe²⁺ resorbiert. Da Nahrungseisen jedoch überwiegend in der dreiwertigen Form vorliegt, muss es, abgesehen vom Häm-gebundenen Fe²⁺-Anteil, zunächst z.B. durch Ascorbinsäure (Vitamin C) reduziert werden, was jedoch bei üblicher Ernährung nur zu einem geringen Anteil gelingt. Resorptionsfördernd sind ein hoher Anteil an Fe²⁺ bei fleischreicher Ernährung sowie ein saures reduzierendes Milieu bei großem Obstund Gemüsekonsum. Resorptionshemmend wirken eine einseitige, insbesondere vegetarische Ernährung, die Bildung nicht absorbierbarer eisenhaltiger Komplexe bei großem Kaffee-, Tee-, oder Limonadenkonsum, sowie Vitamin-C-Mangel. Der Mensch kann das Vitamin nicht selbst produzieren. Die Speicherkapazität des Vitamins ist beim Menschen sehr gering, so dass dieser Stoff permanent zugeführt werden muss. Viele Früchte und Gemüse enthalten Ascorbinsäure. Allerdings ist Vitamin C nicht sehr stabil, deshalb treten schon bei der Lagerung erhebliche Verluste auf. Bei längerem Kochen wird das wasserlösliche Vitamin ebenso aus den Lebensmitteln ausgeschwemmt oder inaktiviert, ebenso wie beim Aufwärmen, Warmhalten oder Wässern.

Um die Vitamin C-Zufuhr zu optimieren und in der Konsequenz auch die Eisenresorption zu verbessern, ist daher eine Kombination von Eisen und Vitamin C in Nahrungsergänzungsmitteln und diätetischen Lebensmitteln sehr sinnvoll. Außerdem fördert Ascorbinsäure die Wundheilung, hemmt die Nitrosaminbildung und ist an antioxidativen Vorgängen beteiligt. Einflüsse auf die Abwehrkräfte. (Lee SH et al., 2001, Calderon PB et al., 2002, Pinnell SR, 2003) werden diskutiert.

Der tägliche Bedarf von Vitamin C wird heute zunehmend kontrovers diskutiert. Als Empfehlung der Deutschen Gesellschaft für Ernährung (DGE) für den täglichen Bedarf an Vitamin C gilt ab März 2000 für Erwachsene ein Bedarf von 100 mg und für Stillende und Raucher von 150 mg.

Vitamin B12 (Cobalamin) ist an vielen Stoffwechselvorgängen beteiligt. Ein Mangel führt zur Blutarmut und zu krankhaften Veränderungen von Haut und Schleimhäuten. Eine normale Nervenzellfunktion benötigt ausreichende Spiegel von Vitamin B12. Es spielt auch eine wesentliche Rolle bei der Überführung der Speicher- und Transportformen der Folsäure in ihre Wirkform und ist unentbehrlich für die normale Erythropoese (Bildung von roten Blutkörperchen) und für die Nervenzellfunktion.

Folsäure ist ein wichtiges Coenzym, ist oft auf die Anwesenheit von Vitamin B12 angewiesen und am Stoffwechsel von Proteinen (Eiweißen) und Nukleinsäuren (DNS) beteiligt. Folsäure ist auch für die Blutbildung erforderlich. Die Beteiligung an der Synthese zu einem Vorstufenprodukt der DNS gehört zu den wichtigsten Aufgaben der Folsäure. Es kann zu indirekten Mangelerscheinungen an Folsäure kommen, wenn Cobalamin nicht in ausreichenden Mengen vorhanden ist. Die Deutsche Gesellschaft für Ernährung (DGE) empfiehlt eine Zufuhr von 400 µg Folsäure pro Tag für Jugendliche und Erwachsene. Wer zuwenig Folsäure aufnimmt, kann unter Müdigkeit, Schlafstörungen, Schleimhautveränderungen oder Unfruchtbarkeit leiden. 19- bis 25-jährige Frauen nehmen durchschnittlich nur zwei Drittel der von der DGE empfohlenen Menge an Folsäure auf. Eine besondere Bedeutung hat Folsäure für Schwangere: Bereits in den ersten Schwangerschaftswochen ist eine ausreichende Folsäureversorgung mit entscheidend für einen optimalen Schwangerschaftsverlauf und die Entwicklung des Kindes. Mögliche Folgen einer Unterversorgung schwangerer Frauen mit Folsäure sind: schwere Missbildungen des Kindes (Neuralrohrdefekt) oder sogar eine Fehlgeburt.

Um 25 µg Vitamin B12 aufzunehmen, müssten z.B. täglich 50 g frische Leber, 500 g Forelle, 300 g Hering oder 600 g Rindfleisch gegessen werden.

Um 600 µg Folsäure mit der Nahrung zu substituieren, müssten täglich entweder 200 g frische Leber, 300 g Sojabohnen, 200 g Kichererbsen oder 400 g Feldsalat verzehrt werden.

Vitamin B6 wird ebenfalls für viele Stoffwechselvorgänge benötigt. Die normale Funktion der Nervenzellen ist von einer ausreichenden Konzentration von Vitamin B6 abhängig. Die physiologische Blutbildung funktioniert nur mit normalen Vitamin B6-Spiegeln.

Die tägliche Menge von 12 mg Vitamin B6 wird mit 300 g Weizenkeimen oder 1200 g Sojabohnen; die Menge von 75 µg Biotin mit 300 g Hühnerei, 250 g Erdnüssen oder 100 g frischer Leber aufgenommen.

Im Stand der Technik sind schon eine ganze Reihe von Eisenpräparaten bekannt, die als Medikamente o.ä. geeignet sein sollen, eine Verbesserung der Eisenaufnahme zu gewährleisten.

Die Ferro-C-Calcium Trinkampullen der Firma Riemser, Deutschland weisen einen Gehalt von 12,5 g Fe²⁺ auf. Weitere Bestandteile umfassen Calciumgluconat (entsprechend 63 mg Ca²⁺) sowie Ascorbinsäure. Als Tagesdosis wird eine Ampulle pro Tag empfohlen. Sie dienen allgemein der Verbesserung des Wohlbefindens. Eine Auffüllung des Eisenspeichers für die genannten, besonders gefährdeten Gruppen gelingt damit nicht.

Die Ferrodix Eisen-Dragees der Firma Duopharm umfassen Eisen(II)-fumarat entsprechend 10 mg Eisen und Eisen(II)-gluconat entsprechend 7,5 mg Eisen, d.h. einen gesamten Eisengehalt von 17,5 mg Eisen sowie 20 mg Ascorbinsäure. Die empfohlene Tagesdosis schwankt zwischen 1 (für Kinder unter 4 Jahren) bis 6 Dragees (während der Stillzeit) pro Tag. Erwachsenen wird eine Miridestdosis von 2 Dragees pro Tag, d.h. 35 mg Eisen empfohlen.

Die Kendural® Fol-500 Depottabletten der Firma Abbott umfassen 329,7 mg Eisen(II)-sulfat H₂O-frei entsprechend 100 mg Eisen, 500 mg Ascorbinsäure und 350 µg Folsäure. Die empfohlene Tagesdosis beträgt eine Depottablette pro Tag.

Die Hämatopan® 50/-100 Dragees der Firma Wolff enthalten u.a. Eisen(II)-sulfat H₂O-frei entsprechend 50/100 mg Eisen sowie 75/150 mg Ascorbinsäure. Die empfohlene Tagesdosis entspricht 50 - 300 mg Eisen.

Die Ferrum Verla® Brausetabletten der Firma Verla weisen einen Eisengehalt von 82,5 mg Fe²⁺ sowie 100 mg Ascorbinsäure auf. Die empfohlene Tagesdosis entspricht 41,25 mg Eisen bei Kindern unter 1½ jahren bis 241,5 mg Eisen.

Die Ferrogamma® Kapseln der Firma Wörwag weisen einen Eisengehalt von 60 mg Fe²⁺ sowie 120 mg Ascorbinsäure auf. Die empfohlene Tagesdosis entspricht 60 bis 120 mg Eisen.

Die Dreisafer® Filmtabletten der Firma GRY weisen einen Eisengehalt von 100 mg Fe²⁺ sowie 300 mg Vitamin C auf. Die empfohlene Tagesdosis entspricht 100 bis 200 mg Eisen.

US 6 562 378 betrifft Nahrungsergänzungsmittel, die neben Eisen und Vitamin C 23 weitere Ergänzungsstoffe enthalten. Das Verhältnis von Eisen zu Vitamin C beträgt 1 zu ca. 2 bis 3,6. Weder ist ein Zusammenhang zwischen der Eisen- und Vitamin C-Konzentration beschrieben noch wird eine tägliche Dosis von Eisen oder Vitamin C offenbart.

US 6 040 333 (siehe auch US 2002/197330) beschreibt Nahrungsergänzungsmittel für Frauen in unterschiedlichen Lebensphasen, die neben Eisen und Vitamin C 13 weitere Ergänzungsstoffe enthalten. Die beschriebenen Mengen an Eisen und Vitamin C reichen jedoch nicht aus, um die bioverfügbare Eisenmenge signifikant zu erhöhen.

US 5 160.728 offenbart allgemein Präparate zur Versorgung mit Eisen und Vitaminen. Die erforderlichen Mengen zur optimalen Resorption von Eisen gehen aus dieser Schrift nicht hervor.

Aufgabe der vorliegenden Erfindung war es daher, eine Zusammensetzung zu finden, die bei möglichst geringem Eisengehalt die genannten Nachteile des Stands der Technik überwindet. Insbesondere sollte es die Zusammensetzung ermöglichen, bei Dauerspendern, aber auch bei weiteren gefährdeten Gruppen wie Kindern und Jugendlichen, menstruierenden Frauen, Schwangeren, Stillenden und Leistungssportlern, den Eisenspeicher wieder aufzufüllen bzw. bei Schwangerschaft oder vegetarischer Ernährung konstant zu halten und gleichzeitig die Verhinderung der genannten Nachteile einer erhöhten Eisenzufuhr mit der Begünstigung der Blutbildung (Blutnachbildung) zu verbinden. Insbesondere sollte diese Zusammensetzung auch dazu geeignet sein, bei Schwangeren einer möglichen Fehlbildung des Fötus vorzubeugen. Weitere, nicht explizit genannte Aufgaben ergeben sich aus dem einleitend zitierten Stand der Technik.

Die vorstehend genannte Aufgabe kann überraschenderweise gelöst werde, indem eine Zusammensetzung mit allen Merkmalen des vorliegenden Patentanspruchs 1 zur Verfügung gestellt wird.

Ein bevorzugtes Ausführungsbeispiel der vorliegenden Erfindung stellt daher ein Präparat für eine ergänzende bilanzierte Diät dar, umfassend die folgenden Bestandteile in einer Menge, die eine tägliche Dosierung über die Nahrung von
a) 5-45 mg Fe²⁺, bevorzugt 15 - 40 mg Fe²⁺, besonders bevorzugt etwa 20 mg Fe²⁺,
b) 50 - 900 mg Vitamin C, bevorzugt 300 - 500 mg Vitamin C, und besonders bevorzugt etwa 400 mg Vitamin C,
   sowie mit Vorteil gegebenenfalls zusätzlich mindestens eine tägliche Dosierung ausgewählt aus der Gruppe bestehend aus
c) 0,5 - 50 mg Vitamin B6, bevorzugt 10 - 40 mg Vitamin B6, besonders bevorzugt 20 - 30 mg Vitamin B6, und am meisten bevorzugt etwa 25 mg Vitamin B6,
d) 50 - 900 µg Folsäure, bevorzugt 150 - 900 µg Folsäure, besonders bevorzugt 300 - 750 µg Folsäure, und am meisten bevorzugt etwa 600 µg Folsäure,
e) 1,5 - 150 µg Biotin, bevorzugt 30 - 100 µg Biotin, und besonders bevorzugt etwa 75 µg Biotin, und
f) 2 - 500 µg Vitamin B12; bevorzugt 25 - 300 µg Vitamin B12, und ganz besonders bevorzugt etwa 250 µg Vitamin B12,
bewirkt.

Somit stellen Eisen und Vitamin C essentielle Bestandteile der erfindungsgemäßen Zusammensetzung dar. Mindestens eine Zutat, ausgewählt aus der Gruppe bestehend aus Vitamin B6, Folsäure, Biotin und Vitamin B12, kann in der angegebenen Tagesdosis ebenfalls enthalten sein. Es ist bevorzugt, wenn alle angegebenen Bestandteil a) bis f) in der Zusammensetzung vorhanden sind.

Präparate, die obige erfindungsgemäße Zusammensetzungen umfassen, werden im folgenden auch erfindungsgemäße ergänzende bilanzierte Diät oder erfindungsgemäßes diätetisches Lebensmittel für besondere medizinische Zwecke genannt, wobei die Begriffe synonym verwendet werden. Insbesondere werden diese Präparate erfindungsgemäß als ergänzende bilanzierte Diät entsprechend der Richtlinie 1999/21/EG verwendet.

Ein weiteres bevorzugtes Ausführungsbeispiel der vorliegenden Erfindung stellt die Verwendung der erfindungsgemäßen ergänzenden bilanzierten Diät zur Verbesserung der Eisenversorgung beim Menschen dar.

Noch ein weiteres bevorzugtes Ausführungsbeispiel der vorliegenden Erfindung stellt die Verwendung der erfindungsgemäßen ergänzenden bilanzierten Diät zur Verbesserung der Blutnachbildung beim Menschen, ebenso wie die Verwendung des erfindungsgemäßen diätetischen Lebensmittels für besondere medizinische Zwecke beim Menschen dar. Insbesondere findet diese ergänzende bilanzierte Diät Verwendung bei der Eisenmangelanämie.

Damit ist die Verwendung der erfindungsgemäßen bilanzierten Diät insbesondere bevorzugt bei einer rein vegetarischen bzw. fleischarmen Kost oder einer Diät zur Gewichtsreduktion, insbesondere aber auch bei hohem Blutverlust z.B. nach einer Blutspende und bei Frauen während der Menstruation. Weiterhin bevorzugte Anwendungsgebiete liegen in der Gabe zur Unterstützung des erhöhten Eisenbedarfs der Frau in der Schwangerschaft und der Stillzeit.

Bevorzugte Ausführungsformen der vorliegenden Erfindung werden auch durch Verfahren zur Herstellung der erfindungsgemäßen ergänzenden bilanzierten Diät, bei denen die Bestandteile a) und b) sowie gegebenenfalls mindestens einem aus c) bis f) der erfindungsgemäßen Zusammensetzung mit einem geeigneten Trägermittel verbunden werden, dargestellt.

Ein weiteres Ausführungsbeispiel der vorliegenden Erfindung betrifft Kapseln umfassend die erfindungsgemäßen Zusammensetzungen ohne weiteres Trägermittel.

Erfindungsgemäße ergänzende bilanzierte Diätprodukte werden erfindungsgemäß auch "diätetische Lebensmittel für besondere medizinische Zwecke" (im Rahmen einer ergänzenden bilanzierten Diät) genannt. Diese können u.a. als Kapseln oder Tabletten verabreicht werden. Die orale Aufnahme des diätetischen Lebensmittels für besondere medizinische Zwecke ist bevorzugt.

Die Herstellung von Kapseln und Tabletten und weiteren Darreichungsformen ist dem Fachmann geläufig, und für die Zwecke der vorliegenden Erfindung ist die gewählte Dosierungsform nicht von Belang.

Die Erfindung stellt daher auch Zusammensetzungen bereit, die die in Anspruch 1 offenbarte Kombination mit einem konventionellen Trägermittel umfassen. Solche Trägermittel sind z.B. feste oder flüssige Füllstoffe, Verkapselungsmaterialien oder Lösungsmittel. Beispiele für Materialien, die als Träger dienen können, sind Zucker wie Lactose, Glucose und Saccharose; Stärke wie Maisstärke und Kartoffelstärke; Cellulose und deren Derivate wie Natriumcarboxy-methylcellulose, Ethylcellulose und Celluloseacetat; pulverisierter Tragacanth; Malz; Gelatine; Talg; Arzneimittelträger wie Kakaobutter und Zäpfchenwachse; Öle wie Erdnußöl, Baumwollsamenöl, Färberdistelöl, Sesamöl, Olivenöl, Maisöl und Sojabohnenöl; Polyole wie Propylenglykol, Glycerin, Sorbitol, Mannitol und Polyethylenglykol; Ester wie Ethyloleat und Ethyllaureat; Agar; Puffermittel wie Magnesiumhydroxid und Aluminiumhydroxid; Alginsäure; Pyrogen-freies Wasser; isotonische Salzlösung; Ringers Lösung, Ethylalkohol und Phosphatpufferlösungen, wie auch andere nichttoxische kompatible Substanzen, die in solchen Formulierungen verwendet werden. Benetzungsmittel, Emulgatoren und Gleitmittel wie Natriumlaurylsulfat und Magnesiumstearat, wie auch Färbemittel, Gleitmittel, Beschichtungsmittel sowie Parftimierungsmittel und Konservierungsstoffe können ebenso in den Zubereitungen vorhanden sein.

Darüberhinaus kann die erfindungsgemäße Kombination auch Bestandteil eines Getränks, z.B. in Form einer Limonade oder eines Sportgetränks, sein.

Dabei kann die Erfindung auch solcherart ausgestaltet sein, dass erfindungsgemäße Präparate im Rahmen einer bilanzierten Diät zur Behandlung von Erkrankungen bei Eisenmangelanämie angewendet werden. Hierbei kann die Konzentration der Bestandteile der erfindungsgemäßen Zusammensetzung in den weiteren Nahrungsbestandteilen Berücksichtigung finden. Insbesondere kann die erfindungsgemäße Zusammensetzung eingesetzt werden, um bei Blutspendern, die regelmäßig mehrmals im Jahr Blut spenden, zum Auffüllen und Konstanthalten des Eisenspiegels zu dienen.

Im Rahmen der vorliegenden Erfindung wurde in einer Studie der Eisenhaushalt bei Dauerspendern (Frauen und Männern) unter einer 2-monatigen (Männer) bzw. 3-monatigen (Frauen) Gabe von Placebo (400 mg Vit. C, 25 µg B12, 12 mg B6, 75 µg Biotin sowie 600 µg Folsäure), 20 mg Eisen (+ o.g. Zusammensetzung des Placebos) oder 40 mg Eisen (+ o.g. Zusammensetzung des Placebos) verglichen. Die Substitution von 40 mg ersetzt den aktuellen Verlust der Eisenspeicher und füllt frühere Defizite wieder auf, dies wird mit der 20 mg-Substitution in abgeschwächter Form ebenfalls erreicht.

Es überraschte, dass mit der erfindungsgemäßen Zusammensetzung mit so geringen Dosierungen von 20 mg/Tag Fe²⁺, und teilweise von allerhöchstens 40 mg/Tag Fe²⁺ eine vollständige Substitution des Eisenspeichers in kurzer Zeit (2 - 3 Monate) erreicht werden konnte.

In einer weiteren offenen Untersuchung wurde an 100 Blutspendern getestet, ob bereits eine einmonatige Gabe von 20 mg Eisen/Tag, zusammen mit der oben genannten Zusammensetzung des Placebos, den Eisenverlust einer Blutspende kompensieren kann. Völlig überraschend reichte die Gabe von 20 mg Eisen in Kombination mit 400 mg Vitamin C und den oben genannten Vitaminen für den kurzen einmonatigen Zeitraum aus, um den Verlust auszugleichen.

Unter einem ganz besonders bevorzugten Ausführungsbeispiel stellt die vorliegende Erfindung daher Zusammensetzungen bereit, die als bilanzierte Diät eingesetzt werden können, und bei denen Eisen in einer Tagesdosierung zwischen 5 µg und 45 µg zusammen mit einer 10-30fach höheren, bevorzugt 15 bis 25fach höheren und ganz besonders bevorzugt etwa 20fachen höheren Dosierung an Vitamin C verabreicht wird, gegebenenfalls mit den weiteren Bestandteilen c) bis f) entsprechend Anspruch 2.

### BEISPIEL 1

In einer prospektiven dreiarmigen Placebo-kontrollierten Doppelblindstudie wurde geprüft, ob bei einem minimalen Blutspendeabstand von acht Wochen bei Männern bzw. zwölf Wochen bei Frauen eine tägliche Eisensubstitution von 20 oder 40 mg Fe²⁺ in der erfindungsgemäßen Zusammensetzung nach Anspruch 1 ausreicht, einen Abfall des Speichereisens der Blutspender zu verhindern. Die Kontrollgruppe erhält keine Eisensubstitution (Placebo; alle Bestandteile der erfindungsgemäßen Zusammensetzung nach Anspruch 1 bzw. 2, aber kein Eisen). Zur Quantifizierung des Speichereisens werden die Serum-Ferritin-, die Serum-Transferrin-Rezeptor- und die Hämoglobin-Konzentration gemessen.

Nach Aufklärung und Einverständniserklärung wurden die Probanden getrennt nach Geschlecht in eine von drei Behandlungsgruppen randomisiert: Die erste Behandlungsgruppe erhielt während des Beobachtungszeitraums 40 mg Fe²⁺ täglich, die zweite Behandlungsgruppe 20 mg Fe²⁺ täglich, die dritte Behandlungsgruppe eine Placebo-Dosierung. Der Beobachtungszeitraum betrug unabhängig vom Geschlecht sechs Monate: Bei den männlichen Spendern wurden vier Vollblutspenden im Abstand von acht Wochen, bei den weiblichen Spendern drei Vollblutspenden im Abstand von zwölf Wochen durchgeführt. Lag bei einem Spendetermin die Hämoglobin-Konzentration unter 13,5 mg/dl bei Männern bzw. unter 12,5 mg/dl bei Frauen, wurde an diesem Termin keine Blutspende durchgeführt. Zu jedem Spendetermin sowie acht bzw. zwölf Wochen nach dem letzten Termin wurden die Zielgröße Serum-Ferritin-Konzentration sowie die explorativen Größen Serum-Transferrin-Rezeptor- und Hämoglobin-Konzentration bestimmt.

Insgesamt wurden 526 Blutspender, 289 Männer und 237 Frauen, eingeschlossen. Die Dropout-Rate war mit 46,0 % (242/526) unerwartet hoch, mehr als drei Viertel der Studienabbrecher (186/242) erschienen bereits zum ersten Kontrolltermin nicht oder gaben an, die Studien-Dosierung nicht protokollgemäß eingenommen zu haben. Die Anzahl der Studienabbrüche ist in den drei Behandlungsgruppen ungleich verteilt (Fishers exakter Test: p = 0.003): Die Anzahl der Studienabbrüche ist in der Behandlungsgruppe mit 40 mg Fe²⁺ am niedrigsten und in der Placebogruppe am höchsten.

Zielgröße der Studie ist Serum-Ferritin-Konzentration. Die Bestimmung der Ferritin-Konzentration im Serum ist die Standardmethode zur Abschätzung des Gesamtkörpereisenbestandes: Eine Ferritin-Konzentration 1 µg/l Ferritin entspricht etwa einem Körpereisenbestand von 8 mg. Eine Ferritin-Konzentration von unter 12 µg/l weist auf einen kompletten Verlust des Speichereisens hin. Die Ferritin-Konzentration ist weitgehend stabil und weist weder zirkadiane noch andere biologische Schwankungen auf. Bei akuten und chronischen Entzündungen, Malignomen und Leberparenchymerkrankungen kann die FerritinKonzentration falsch hoch sein, erniedrigte Ferritin-Konzentrationen sind jedoch immer spezifisch für einen Eisenmangel.

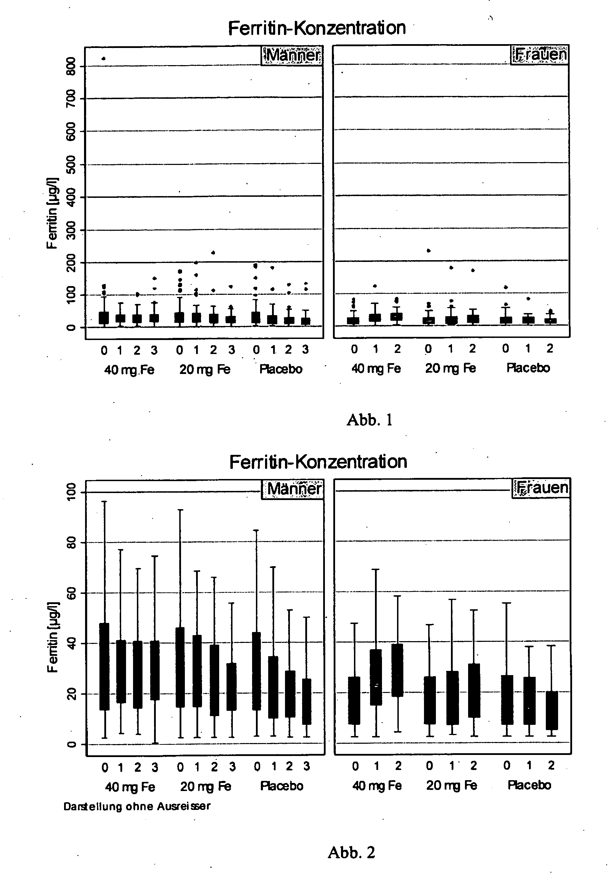

Tabelle 1 zeigt Mittelwerte und Standardabweichungen für die Serum-Ferritin-Konzentration in den drei Behandlungsgruppen in Abhängigkeit von Geschlecht und Untersuchungstermin. In Abbildung 1 und 2 sind die Serum-Ferritin-Konzentrationen als Box-Plots dargestellt, in Abbildung 2 wurde zur besseren Übersichtlichkeit eine Darstellung ohne Ausreißer gewählt.

**Tabelle 1: Mittelwert und Standardabweichung (kursiv und klein gedruckt) der Serum-Ferritin-Konzentration [µg/l] in Abhängigkeit von Dosierung, Geschlecht und Untersuchungstermin.**

| | Untersuchungstermin | Dosierung | | |
|---|---|---|---|---|
| | | 40 mg Fe | 20 mg Fe | Placebo |
| Männer | 0 | 43,4 | 36,6 | 34,9 |
| | | *85,2* | *36,8* | *34,2* |
| | 1 | 31,4 | 33,1 | -27,5 |
| | | *18,8* | *33,3* | *27,9* |
| | 2 | 30,2 | 30,2 | 24,9 |
| | | *20,8* | *31,7* | *24,7* |
| | 3 | 33,0 | 25,0 | 21,4 |
| | | *26,9* | *19,8* | 2*7,5* |
| Frauen | 0 | 20,8 | 19,8 | 20,0 |
| | | *16,6* | *27,8* | *17,9* |
| | 1 | 28,5 | 23,3 | 17,6 |
| | | *19,8* | *27,9* | *14,5* |
| | 2 | 31,4 | 23,5 | 15,1 |
| | | *19,4* | *26,1* | *12,3* |

Für die intent-to-treat-Analyse der Zielgröße wird eine Regressionsanalyse verwendet. Dabei ist zu beachten, dass die Messwerte der einzelnen Probanden an den verschiedenen Untersuchungszeitpunkten nicht unabhängig voneinander sind. Es wird deshalb eine entsprechende Korrektur für Daten-Cluster mit robuster Varianzschätzung ("Huber-White-sandwich estimator of variance") angewendet. Für männliche und weibliche Spender werden unterschiedliche Regressionsmodelle verwendet. Als unabhängige Faktoren werden der Untersuchungszeitpunkt, die Art der Dosierung (40 mg Eisen täglich, 20 mg Eisen täglich, Placebo) und der Ferritin-Ausgangswert eingeschlossen. Interaktionen ersten Grades werden nur in das Modell einbezogen, wenn sie einen signifikanten Effekt (p < 0,05) aufweisen.

Tabelle 2 zeigt das Regressionsmodell für die männlichen, Tabelle 3 für die weiblichen Spender.

**Tabelle 2: Regressionsmodell der Seriun-Ferritin-Konzentration für die männlichen Spender (R² = 0,816)**

| **Parameter** | **Codierung** | **Koeffizient** | **95 %-Konfidenzinterval** | | **P** |
|---|---|---|---|---|---|
| Behandlungsgruppe 1 (40 mg Fe²⁺) | 0 bei Placebo oder 20 mg Fe²⁺ | 0,85 | -1,34 | 3,03 | 0,446 |
| | 1 bei 40 mg Fe²⁺ | | | | |
| Behandlungsgruppe 2 (20 mg Fe²⁺) | 0 bei Placebo oder 40 mg Fe²⁺ | 1,48 | -0,90 | 3,86 | 0,223 |
| | 1 bei 20 mg Fe²⁺ | | | | |
| Untersuchungstermin | 1 bis 4 | -5,04 | -7,26 | -2,81 | < 0,001 |
| Interaktion: Untersuchungstermin x | 0 bei Placebo oder 20 mg Fe²⁺ | 5,95 | 3,11 | 8,79 | <0,001 |
| Behandlungsgruppe 1 (40 mg Fe²⁺) | 1 bis 4 bei 40 mg Fe²⁺ | | | | |
| Interaktion: Untersuchungstermin x | 0 bei Placebo oder 40 mg Fe²⁺ | 3,24 | -0,84 | 6,56 | 0,056 |
| Behandlungsgruppe 2 (20 mg Fe²⁺) | 1 bis 4 bei 20 mg Fe²⁺ | | | | |
| Ferritin-Ausgangswert | Zahlenwert | 0,86 | 0,74 | 0,99 | < 0,001 |
| Konstante | | 3,73 | -0,85 | 8,31 | 0,110 |

**Tabelle 3: Regressionsmodell der Serum-Ferntin-Konzentration für die weiblichen Spender (R² = 0,718)**

| **Parameter** | **Codierung** | **Koeffizient** | **95 %-Konfidenzinterval** | | **P** |
|---|---|---|---|---|---|
| Behandlungsgruppe 1 (40 mg Fe²⁺) | 0 bei Placebo oder 20 mg Fe²⁺ | 0,69 | -0,84 | 2,22 | 0,375 |
| | 1 bei 40 mg Fe²⁺ | | | | |
| Behandlungsgruppe 2 (20 mg Fe²⁺) | 0 bei Placebo oder 40 mg Fe²⁺ | 0,41 | -1,30 | 2,12 | 0,638 |
| | 1 bei 20 mg Fe²⁺ | | | | |
| Untersuchungstermin | 1 bis 3 | -1,78 | -4,04 | 0,47 | 1,21 |
| Interaktion: Untersuchungstermin x | 0 bei Placebo oder 20 mg Fe²⁺ | 7,86, | 4,68 | 11,04 | <0,001 |
| Behandlungsgruppe 1 (40 mg Fe²⁺) | 1 bis 3 bei 40 mg Fe²⁺ | | | | |
| Interaktion: Untersuchungstermin x | 0 bei Placebo oder 40 mg Fe²⁺ | 3,40 | -0,21 | 6,58 | 0,037 |
| Behandlungsgruppe 2 (20 mg Fe²) | 1 bis 3 bei 20 mg Fe²⁺ | | | | |
| Ferritin-Ausgangswert | Zahlenwert | 0,80 | 0,74 | 0,85 | < 0,001 |
| Konstante | | 4,40 | 3,02 | 5,78 | < 0,001 |

Beide Regressionsmodelle erklären die Daten sehr gut: 82 % bzw. 72 % der Varianz der Serum-Ferntin-Konzentrationen werden durch das jeweilige Regressionsmodell erklärt.

Bei den männlichen Spendern zeigt sich in der Placebo-Gruppe eine signifikante Abnahme der Ferritin-Konzentration um jeweils etwa 5,0 µg/l zwischen zwei Untersuchungsterminen (p < 0,001). Der Abfall der FerritinKonzentration ist tendenziell, aber nicht signifikant geringer unter Substitution mit 20 mg Eisen täglich (p = 0,056). Unter Substitution mit 40 mg Eisen täglich hingegen findet sich ein davon signifikant abweichendes Verhalten mit einem Anstieg der Ferritin-Konzentration um jeweils etwa 0,9 µg/l zwischen zwei Untersuchungsterminen (p < 0,001). Daneben wird erwartungsgemäß die FerritinKonzentration vom Ausgangswert vor der ersten Spende beeinflusst (p < 0,001).

Bei den weiblichen Spendern zeigt sich in der Placebo-Gruppe keine signifikante Änderung der Ferritin-Konzentration zwischen zwei Untersuchungsterminen. Hingegen findet sich bereits unter Substitution mit 20 mg Eisen täglich ein schwach signifikanter Anstieg der Ferritin-Konzentration um jeweils etwa 3,4 µg/l zwischen zwei Untersuchungsterminen (p = 0,037). Unter Substitution mit 40 mg Eisen täglich zeigt sich ein signifikanter Anstieg der Ferritin-Konzentration um jeweils etwa 7,9 µg/l zwischen zwei Untersuchungsterminen (p < 0,001). Auch bei den weiblichen Spendern wird die Ferritin-Konzentration vom Ausgangswert vor der ersten Spende beeinflusst (p < 0,001).

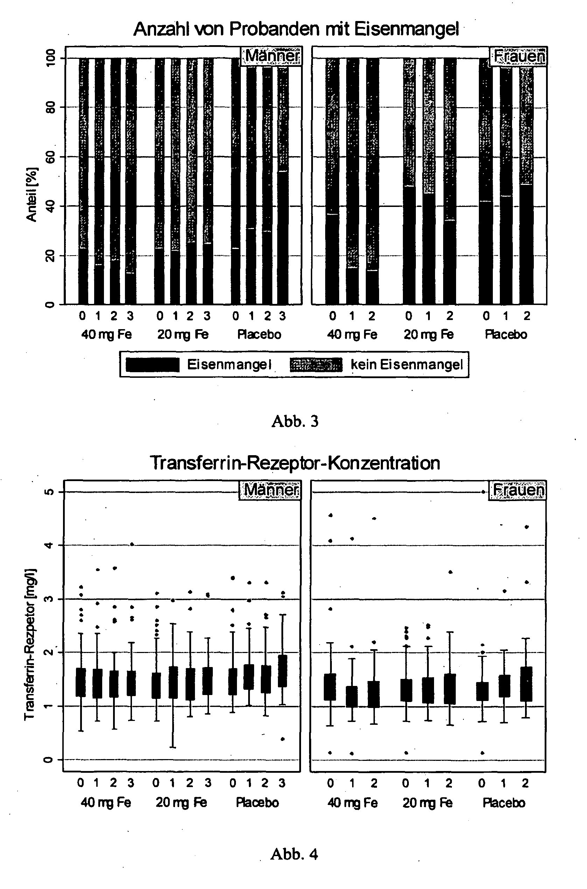

Tabelle 4 und Abbildung 3 zeigen die Häufigkeit eines Speichereisenmangels (Serum-Ferritin < 12 µg/l) in den Behandlungsgruppen. Es finden sich vergleichbare Ergebnisse zur Analyse der Ferritin-Konzentration: Unabhängig vom Geschlecht steigt in der Placebo-Gruppe die Anzahl an Spendern mit Speichereisenmangel mit der Zahl der Spenden an, während sie unter Substitution mit 40 mg Eisen täglich abnimmt. Während bei den Frauen jedoch die Anzahl der Spender mit Speichereisenmangel bei Substitution mit 20 mg Eisen täglich abnimmt, bleibt sie bei den Männern nahezu unverändert.

**Tabelle 4: Häufigkeit [%] eines Speichereisenmangels (Ferritin < 12 µg/l) in Abhängigkeit von Dosierung, Geschlecht und Untersuchungstermin**

| | Untersuchungstermin | Dosierung | | |
|---|---|---|---|---|
| | | 40 mg Fe | 20 mg Fe | Placebo |
| Männer | 0 | 22,9 | 22,6 | 22,9 |
| | 1 | 16,2 | 21,8 | 30,9 |
| | 2 | 17,9 | 25,4 | 29,8 |
| | 3 | 13,0 | 24,5 | 53,8 |
| Frauen | 0 | 36,7 | 48,1 | 41,8 |
| | 1 | 15,2 | 45,1 | 44,2 |
| | 2 | 14,0 | 34,1 | 48,7 |

Als explorative Größen wurden die Serum-Transferrin-Rezeptor- und die Hämoglobin-Konzentration gemessen.

Der Transferrin-Rezeptor ist ein transmembranes Protein, das Transferrin an der Zelloberfläche bindet und in den Intrazellularraum transportiert. Der lösliche Transferrin-Rezeptor im Blut ist ein Bruchstück dieses Proteins und korreliert eng mit der Gesamtmenge des Organismus an Transferrin-Rezeptor. Er ist empfindlicher Indikator für die Eisenversorgung der Erythropoese, allerdings führt erst ein Eisenmangel im Stadium der eisendefizitären Hämatopoese und der Eisenmangelanämie zu einer vermehrten Expression des Transferrin-Rezeptors. Zur Abschätzung des Gesamtkörpereisens ist die Serumkonzentration des löslichen Transferrin-Rezeptors deshalb weniger geeignet als die FerritinKonzentration. Die Hämoglobin-Konzentration fällt erst bei manifestem Eisenmangel mit dem Vollbild der Eisenmangelanämie unter die Normgrenze ab.

Tabelle 5 und Abbildung 4 zeigen die Serum-Transferrin-RezeptorKonzentration in den Behandlungsgruppen.

**Tabelle 5: Mittelwert und Standardabweichung (kursiv und klein gedruckt) der Serum-Transferrin-Rezeptor-Konzentration [mg/l] in Abhängigkeit von Dosierung, Geschlecht und Untersuchungstermin**

| | Untersuchungstermin | Dosierung | | |
|---|---|---|---|---|
| | | 40 mg Fe | 20 mg Fe | Placebo |
| Männer | 0 | 1,50 | 1,45 | 1,53 |
| | | *0,49* | *0,45* | *0.50* |
| | 1 | 1,47 | 1,46 | 1,61 |
| | | *0,49* | *0,44* | *0.45* |
| | 2 | 1,50 | 1,47 | 1,60 |
| | | *0,51* | *0,45* | *0,52* |
| | 3 | 1,52 | 1,52 | 1,67 |
| | | *0,55* | *0,47* | *0,53* |
| Frauen | 0 | 1,41 | 1,36 | 1,34 |
| | | *0,61* | *0,42* | *0,53* |
| | 1 | 1,26 | 1,36 | 1,40 |
| | | *0,49* | *0,41* | *0,42* |
| | 2 | 1,29 | 1,35 | 1,55 |
| | | *0,54* | *0,49* | *0,66* |

Die Serum-Transferrin-Rezeptor-Konzentration verhält sich umgekehrt wie die Serum-Ferritin-Konzentration, d.h. sie weist leicht ansteigende Werte in der Placebo-Gruppe auf. Fallende Werte zeigen sich nur bei den weiblichen Spendern unter Substitution mit 40 mg Eisen täglich.

Die Studienergebnisse zeigen, dass bei den Männern eine Substitution von 40 mg Fe²⁺ über acht Wochen, bei den Frauen eine Substitution mit 20 mg Fe²⁺ über zwölf Wochen ausreicht, um den Abfall des Speichereisen durch die Blutspende nicht nur zu kompensieren, sondern in einem Teil der Fälle sogar einen Speichereisenmangel auszugleichen.

### BEISPIEL 2

In einer offenen Studie wurden 126 Blutspender, 67 Männer und 59 Frauen, mit einer Kombination aus 20 mg Fe²⁺ und 400 mg Vitamin C über vier Wochen behandelt. Die Dropout-Rate war mit 27,8 % (35/126) sehr hoch. Die Gründe für den Studienabbruch liegen derzeit nicht vor, so dass eine Dropout-Analyse nicht möglich ist. Ausgewertet wurden 91 Probanden, davon 52 Männer und 39 Frauen.

9,6 % (5/52) der ausgewerteten Männer und 7,8 % (3/39) der ausgewerteten Frauen wiesen beim 2. Termin eine kapilläre Hämoglobin-Konzentration unter dem zulässigen Grenzwert auf und wurden deshalb von einer erneuten Blutspende zurückgestellt. Bei 7/8 dieser Probanden erfolgte keine Bestimmung der Ferritin- und Transferrin-Rezeptor-Konzentration. Im Folgenden wurde eine Auswertung auf Basis der vorhandenen Daten durchgeführt.

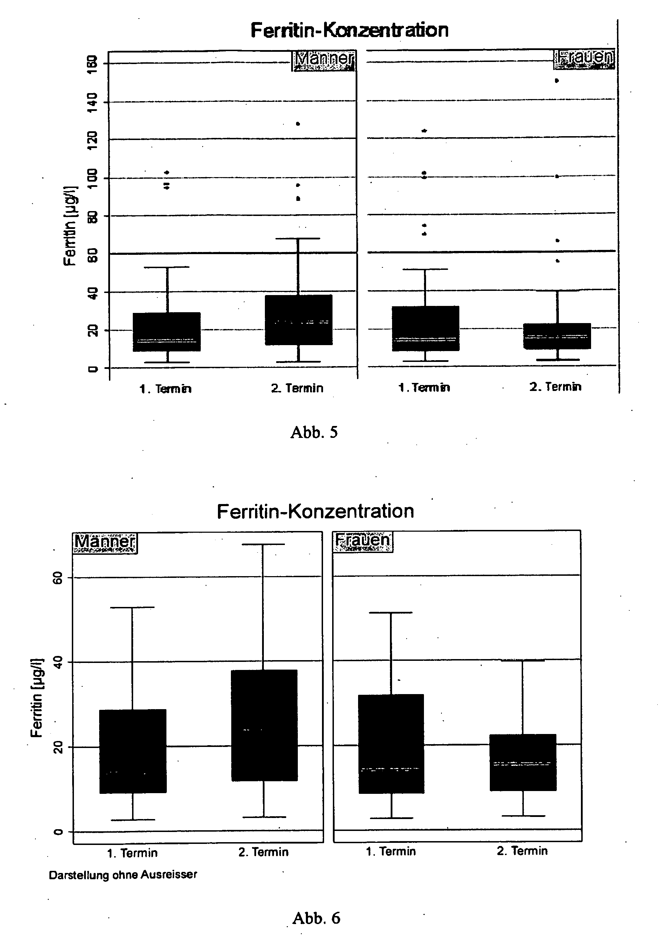

Tabelle 6 zeigt Mittelwerte und Standardabweichungen für die Serum-Ferritin-Konzentration getrennt für die männlichen und weiblichen Probanden. In Abbildungen 5 und 6 ist die Serum-Ferritin-Konzentration als Box-Plots dargestellt, in Abbildung 6 wurde zur besseren Übersichtlichkeit eine Darstellung ohne Ausreißer gewählt.

**Tabelle 6: Mittelwert und Standardabweichung (kursiv und klein gedruckt) der Serum-Ferritin-Konzentration in Abhängigkeit von Geschlecht und Untersuchungstermin**

| | Untersuchungstermin | Ferritin [µg/l] |
|---|---|---|
| Männer | 1 | 23,0 |
| | | *23,2* |
| | 2 | 28,5 |
| | | *25,0* |
| Frauen | 1 | 26,9 |
| | | *29,3* |
| | 2 | 23,7 |
| | | *28,7* |

Die konfirmatorische Datenanalyse mit dem t-Test für verbundene Stichproben ergibt einen signifikanten Anstieg der Ferntin-Konzentration um im Mittel 5,8 µg/l (p = 0,0101) bei den männlichen Probanden, jedoch keine signifikante Änderung bei den weiblichen Probanden (p = 0,2727).

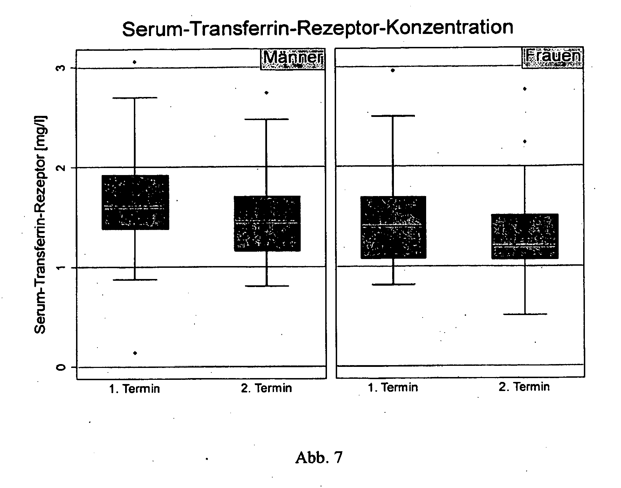

Tabelle 7 und Abbildung 7 zeigen die statistischen Kennwerte und das Boxplot für die Serum-Transferrin-Rezeptor-Konzentration. Es findet sich ein signifikanter Abfall der Transferrin-Rezeptor-Konzentration um im Mittel 0,18 mg/l bei den männlichen und 0,15 mg/l bei den weiblichen Probanden (p = 0,0026 bzw. p = 0,0045).

**Tabelle 7: Mittelwert und Standardabweichung (kursiv und klein gedruckt) der Serum-Transferrin-Rezeptor-Konzentration in Abhängigkeit von Geschlecht und Untersuchungstermin**

| | Untersuchungstermin | Transferrin-Rezeptor [mg/l] |
|---|---|---|
| Männer | 1 | 1,69 |
| | | *0,51* |
| | 2 | 1,48 |
| | | *0,44* |
| Frauen | 1 | 1,44 |
| | | *0,45* |
| | 2 | 1,30 |
| | | *0,44* |

## Patentansprüche

1. Verwendung einer Zusammensetzung umfassend Fe²⁺ und Vitamin C im Verhältnis der Gewichtsteile von etwa 1 zu 20 zur Herstellung eines Präparates für eine ergänzende bilanzierte Diät zur Verbesserung der Eisenversorgung beim Menschen, zur Behandlung von Eisenmangelanämie oder zur Verbesserung der Blutnachbildung beim Menschen,
wobei das Präparat in einer Dosis von täglich etwa 20 mg Fe²⁺ und 400 mg Vitamin C verabreicht wird.

2. Verwendung nach Anspruch 1, wobei die Zusammensetzung so dosiert wird, dass täglich
a) 0,5 - 50 mg Vitamin B6, bevorzugt 10 - 40 mg Vitamin B6, besonders bevorzugt 20-30 mg Vitamin B6, und am meisten bevorzugt etwa 25 mg Vitamin B6, und/oder
b) 50 - 900 µg Folsäure, bevorzugt 150 - 900 µg Folsäure, besonders bevorzugt 300 - 750 µg Folsäure, und am meisten bevorzugt etwa 600 µg Folsäure, und/oder
c) 1,5 - 150 µg Biotin, bevorzugt 30 - 100 µg Biotin, und besonders bevorzugt etwa 75 µg Biotin, und/oder
d) 2 - 500 µg Vitamin B12, bevorzugt 25 - 300 µg Vitamin B12, und ganz besonders bevorzugt etwa 250 µg Vitamin B12,
durch die Zusammensetzung zugeführt werden.

3. Verwendung nach Anspruch 1 zur Verbesserung der Eisenversorgung bei rein vegetarischer bzw. fleischarmer Kost oder einer Diät zur Gewichtsreduktion, bei hohem Blutverlust, z.B. nach einer Blutspende und bei Frauen während der Menstruation, sowie zur Unterstützung des erhöhten Eisenbedarfs der Frau in der Schwangerschaft und der Stillzeit.

4. Verwendung nach einem der Ansprüche 1 - 3, wobei die Zusammensetzung in Verbindung mit einem geeigneten Trägermittel vorliegt.

## Claims

1. Use of a compound comprising Fe²⁺ and vitamin C in a ratio of parts by weight of approx.
1 to 20 for the production of a preparation for a supplementary balanced diet for improving the iron supply in human beings, for the treatment of iron deficiency anaemia or for improving blood reproduction in human beings, wherein the preparation is administered in a daily dose of approx. 20 mg Fe²⁺ and 400 mg vitamin C.

2. The use according to claim 1, wherein the compound is dosed in such a way that daily
a) 0.5 - 50 mg vitamin B6, preferably 10 - 40 mg vitamin B6, particularly preferably 20 - 30 mg vitamin B6, and most preferably approx. 25 mg vitamin B6, and/or
b) 50 - 900 µg folic acid, preferably 150 - 900 µg folic acid, particularly preferably 300 - 750 µg folic acid, and most preferably approx. 600 µg folic acid, and/or
c) 1.5 - 150 µg biotin, preferably 30 - 100 µg biotin, and particularly preferably approx. 75 µg biotin, and/or
d) 2 - 500 µg vitamin B12, preferably 25 - 300 µg vitamin B12, and very particularly preferably approx. 250 µg vitamin B12,
are supplied through the compound.

3. The use according to claim 1 for improving the iron supply in purely vegetarian food or food containing little meat or a diet for weight reduction, in the case of high blood loss, e.g. after donating blood, and in the case of women during menstruation, as well as to assist with the raised iron requirement of women during pregnancy and breast-feeding.

4. The use according to any one of claims 1 - 3, wherein the compound is present in combination with a suitable carrier.

## Revendications

1. Utilisation d'une composition comprenant Fe²⁺ et de la vitamine C dans un rapport des parties pondérales d'environ 1 à 20 à la production d'une préparation pour un régime de complémentation équilibré pour l'amélioration de l'approvisionnement en fer chez l'Homme, pour le traitement de l'anémie par carence en fer ou pour l'amélioration de l'hématopoïèse chez l'Homme,
la préparation étant administrée à une dose journalière d'environ 20 mg de Fe²⁺ et d'environ 400 mg de vitamine C.

2. Utilisation selon la revendication 1, la composition étant dosée de telle sorte que, par jour,
a) 0,5 à 50 mg de vitamine B6, de préférence 10 à 40 mg de vitamine B6, encore plus préférablement 20 à 30 mg de vitamine B6 et de façon particulièrement préférée environ 25 mg de vitamine B6 et/ou
b) 50 à 900 µg d'acide folique, de préférence 150 à 900 µg d'acide folique, encore plus préférablement 300 à 750 µg d'acide folique et de façon particulièrement préférée environ 600 µg d'acide folique, et/ou
c) 1,5 à 150 µg de biotine, de préférence 30 à 100 µg de biotine, de façon particulièrement préférée environ 75 µg de biotine, et/ou
d) 2 à 500 µg de vitamine B12, de préférence 25 à 300 µg de vitamine B12 et de façon particulièrement préférée environ 250 µg de vitamine B12,
sont administrés par la composition.

3. Utilisation selon la revendication 1 pour l'amélioration de l'approvisionnement en fer dans le cas d'un régime purement végétarien ou pauvre en viande, ou d'un régime en vue d'une perte de poids, en cas de forte perte de sang, par exemple après un don du sang et pendant la menstruation chez les femmes, ainsi que pour soutenir les besoins accrus en fer de la femme enceinte ou allaitante.

4. Utilisation selon l'une quelconque des revendications 1 à 3, la composition se présentant en association avec un support approprié.
